# FASCICULE DE BREVET EUROPEEN

(11) **EP 2 575 648 B1**
(45) Date de publication et mention de la délivrance du brevet: **16.08.2017**
(21) Numéro de dépôt: 11725982.0
(22) Date de dépôt: 26.05.2011
(51) Int. Cl.: A61B 17/3203, A61M 5/148, A61M 5/168, A61M 3/02, A61B 17/00

(54) **GÉNÉRATEUR DE JETS PULSES MOYENNE ET HAUTE PRESSION D'UN LIQUIDE EN VUE D'APPLICATIONS MÉDICALES ET CHIRURGICALES**
GEPULSTER MITTEL- UND HOCHDRUCK-FLÜSSIGKEITSSTRAHLGENERATOR FÜR MEDIZINISCHE UND CHIRURGISCHE ZWECKE
PULSED MEDIUM- AND HIGH-PRESSURE LIQUID JET GENERATOR FOR MEDICAL AND SURGICAL USES

(30) Priorité: 27.05.2010 FR 1002244
(43) Date de publication de la demande: 10.04.2013
(73) Titulaire: Nestis, 38100 Grenoble (FR)
(72) Inventeur: GONON, Bertrand, F-69360 Ternay (FR)
(74) Mandataire: Vossius & Partner Patentanwälte Rechtsanwälte mbB
(86) Numéro de dépôt international: PCT/IB2011/052291
(87) Numéro de publication internationale: WO 2011/148333

(56) Documents cités:
- EP-A1- 0 098 893
- WO-A1-97/49441
- WO-A2-02/20073
- US-A- 5 163 909

## Description

La présente invention concerne un générateur d'impulsions liquides de moyenne et de haute pression ou de jets pulsés prévu pour divers travaux médicaux et chirurgicaux à partir d'un liquide dosé ou non stérile ou de traitement ou un support liquide dosé ou non contenant des cellules ou des microorganismes ou pour mettre en oeuvre un procédé de traitement ou d'injection.

Plus particulièrement, l'invention concerne un générateur de jets pulsés de moyenne et de haute pression dans lequel au moins une poche souple de liquide médical à usage unique est mise sous pression contrôlée au moyen d'un gaz comprimé et dans lequel l'éjection du liquide hors d'une ou des poches souples de liquide médical est pulsée et séquencée au moyen d'un séquenceur hydraulique.

Les applications de ce générateur sont multiples. Elles se trouvent notamment mais non exclusivement dans les domaines de la chirurgie, de l'endoscopie et de la biothérapie.

Actuellement, les générateurs de jets pulsés haute pression utilisés dans les domaines aussi bien médical que chirurgical ont plusieurs applications. On connaît déjà plusieurs types de générateurs de jets pulsés. Ceux comparables à celui de l'invention comportent une poche souple d'un liquide médical placée dans une enceinte sous pression de laquelle il sort sous pression constante et contrôlée.

Dans les brevets suivants US2004097889A, US5776104A, US60831891, US5163909A et WO9428807A, sont décrits des générateurs de ce type dans lesquels le liquide médical sous pression passe à l'extérieur de l'enceinte dans un séquenceur hydraulique formant un jet pulsé délivré par une pièce à main d'intervention. Le document EP 0 098 893 A1 divulgue les caractéristiques du préambule de la revendication 1 ci-après. Ce type de séquenceur hydraulique ne permet habituellement pas de créer un jet pulsé présentant des impulsions de liquide de forme en créneaux parfaitement carrés ou rectangulaires, mais crée plutôt un jet pulsé dont les impulsions de pression sont de forme ondulatoire au mieux pseudo impulsionnel à flancs inclinés. En outre, les impulsions obtenues par le jet pulsé sont rarement parfaitement régulières et peuvent varier en intensité ou en période. Ceci est particulièrement désavantageux pour certaines applications médicales ou chirurgicales pour lesquelles le générateur de jets pulsés est utilisé pour un travail précis dépendant de la forme des impulsions de pression. Il en est ainsi notamment dans le domaine chirurgical de la revascularisation transmyocardique où la forme, l'intensité, la durée et la périodicité des pulsations du jet sous pression déterminent la taille et la profondeur des perforations réalisées dans la paroi du coeur.

Le but de l'invention est de fournir un générateur de jets pulsés de moyenne et de haute pression capable de générer des trains de jets pulsés formés d'une succession d'impulsions de pression en forme de créneaux à flancs raides, trains pour lesquels l'intensité, la durée et la périodicité des impulsions de liquide sous pression sont réglables et contrôlées à partir d'un générateur dont tous les organes sont incorporés à l'enceinte haute pression contenant les poches de liquide.

Plus particulièrement, l'invention a pour but de permettre un contrôle simple, rapide et fiable de l'intensité, de la durée et de la périodicité des impulsions de liquide sous pression, dans un générateur de jets pulsés haute pression compact, c'est-à-dire d'encombrement réduit, et d'un coût plus faible que celui des dispositifs existants.

Avantageusement, notamment pour des raisons de modularité, de polyvalence et de sécurité, un tel générateur de jets pulsés haute pression permet l'utilisation d'au moins une poche souple de liquide médical, et se trouve entièrement isolé électriquement, et fonctionne aussi bien en mode pulsé qu'en mode continu.

Pour résoudre ces différents problèmes techniques, le générateur de jets pulsés de moyenne et de haute pression selon l'invention comprend les moyens suivants :
- une enceinte haute pression, dont la pression interne est obtenue à partir d'une source de gaz sous pression et régulée par un dispositif de contrôle de la pression agissant sur une réserve de liquide présente dans un contenant souple ;
- au moins un connecteur pour poches souples de liquide médical présentant chacun un conduit intérieur de raccordement pour le liquide médical, en communication fluidique avec une sortie d'une poche souple, ces connecteurs étant prévus dans l'enceinte haute pression ;

- une chaîne hydraulique interne à l'enceinte comprenant en série, un conduit de raccordement avec le contenant souple, un ensemble modulateur ou séquenceur hydraulique, un tronçon de sortie de l'ensemble modulateur hydraulique raccordé à un conduit extérieur de liaison ;
   pour l'alimentation d'un instrument chirurgical de travail, d'exploration ou de traitement ou d'un cathéter,
   le séquenceur hydraulique comprenant :
   - une réserve tampon momentanée dosée de liquide sous la forme d'une enveloppe déformable souple, d'un tube souple ou d'un composant souple comprimable, bulbe, ballonnet ou autre élément souple de petite contenance monté en série dans la liaison hydraulique,
   - au moins un obturateur hydraulique, mais de préférence deux de part et d'autre de la liaison hydraulique souple, ou du composant comprimable, ce ou ces obturateurs hydrauliques faisant partie du séquenceur hydraulique prévu dans l'enceinte haute pression,
   - un moyen de commande alternative en ouverture et en fermeture du ou des obturateurs hydrauliques ;
- un moyen de raccordement étanche pour alimenter au moins une pièce à main ou un cathéter d'intervention, d'exploration ou de traitement suivi d'un conduit extérieur de liaison avec cette pièce à main ou ce cathéter.

Dans ce dispositif, lorsque l'enceinte est mise sous haute pression, les différents moyens qui s'y trouvent logés sont exposés à la haute pression. Ainsi, la ou les poche(s) souple(s) contenant le liquide médical subit ou subissent la pression intérieure.

Lorsque le moyen de commande ouvre l'obturateur amont des obturateurs hydrauliques situés sur la liaison hydraulique souple, le liquide provenant de la poche souple raccordée à cette liaison hydraulique souple vient remplir l'enveloppe déformable souple.

Lorsque le moyen de commande ouvre l'obturateur hydraulique aval situé sur la liaison hydraulique souple de l'enveloppe déformable souple, le liquide contenu dans celle-ci et éventuellement dans la réserve souple momentanée est éjecté et arrive sous la pression de l'enceinte dans le dispositif d'utilisation situé à l'extérieur de l'enceinte par le conduit extérieur de liaison à une pièce à main ou un cathéter en vue du travail médical ou chirurgical.

L'utilisation préférée d'obturateurs hydrauliques de type tout-ou-rien permet d'obtenir un jet pulsé formé d'impulsions liquides en formes de créneaux à flancs raides, tandis que le moyen de commande permet un contrôle simple, rapide et fiable de l'intensité, de la durée et de la périodicité des pulsations des jets sous pression.

Les principaux éléments constitutifs de l'invention étant tous logés dans une même enceinte, le générateur de jets pulsés moyenne et haute pression selon l'invention présente un encombrement réduit. Ces différents éléments constitutifs étant en outre tous soumis à la même pression, il peut s'agir de composants classiques notamment prévus pour les basses pressions, ce qui réduit le coût du générateur.

D'autres caractéristiques et avantages de l'invention apparaîtront à la lecture de la description détaillée qui va suivre, description faite en référence aux dessins annexés, dans lesquels :
- la figure 1 est une vue schématique d'un générateur de jets pulsés haute pression selon un premier mode de réalisation de l'invention prévu pour l'utilisation d'une seule poche souple de liquide médical ; et
- la figure 2 est une vue schématique d'un générateur de jets pulsés haute pression selon un second mode de réalisation de l'invention prévu pour l'utilisation de deux poches souples de liquide médical, la deuxième poche étant représentée en traits discontinus car optionnelle.

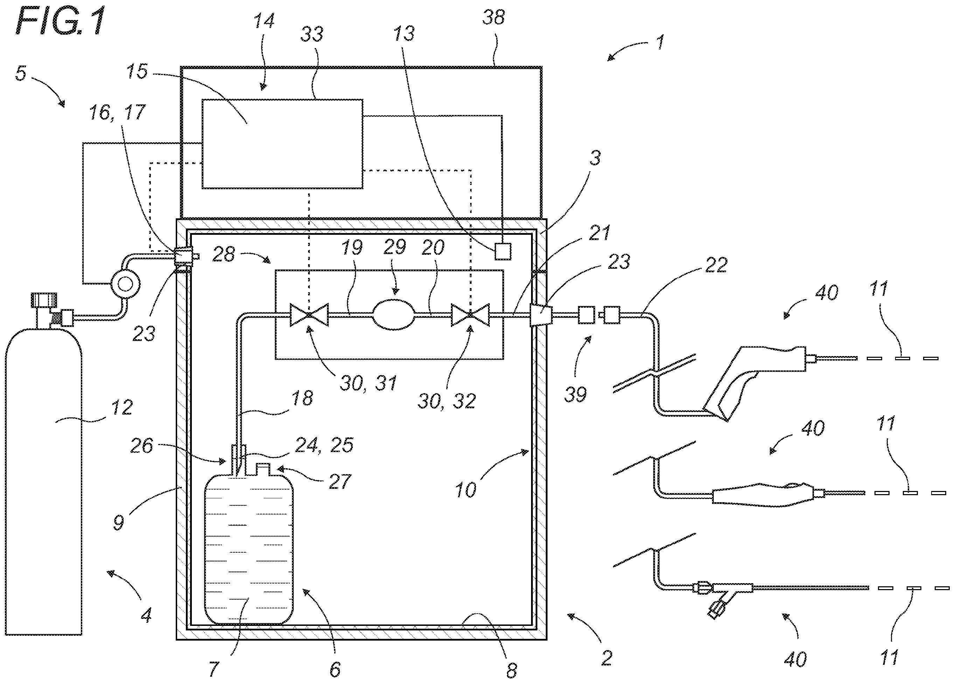

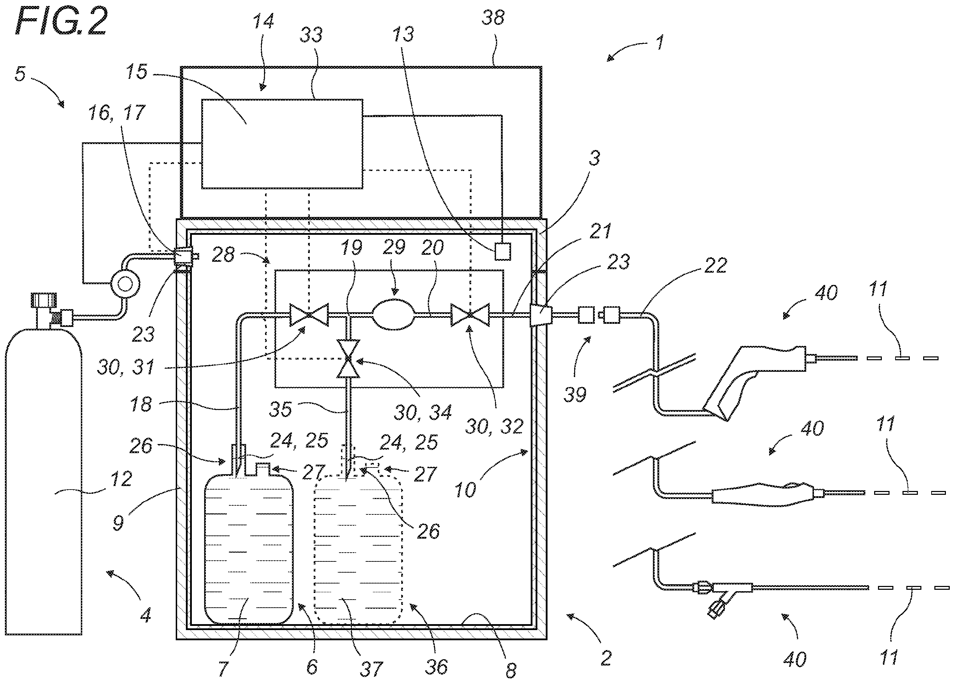

Le générateur de jets pulsés haute pression selon la présente invention va maintenant être décrit de façon détaillée en référence aux figures 1 et 2. Les éléments équivalents représentés sur les différentes figures porteront les mêmes références numériques.

On définira dans la suite de cette description les notions d'amont et d'aval en fonction du sens d'écoulement normal du ou des liquides médicaux utilisés dans le générateur de l'invention, ces liquides s'écoulant depuis les poches souples vers le conduit de sortie après avoir traversé la liaison hydraulique souple et l'enveloppe déformable souple du séquenceur hydraulique.

Le générateur de jets pulsés haute pression 1 selon l'invention comprend une enceinte haute pression 2 obturée par une pièce de fermeture 3, couvercle ou autre, pouvant être ouverte et refermée pour l'accès à son intérieur. La pièce de fermeture 3 de l'enceinte haute pression 2 est préférentiellement un couvercle équipé d'un joint de type autoclave ou venant porter sur un tel joint.

L'enceinte haute pression 2 est étanche et prévue pour résister à de hautes pressions internes, par exemple supérieures à 50 bars, préférentiellement supérieures à 30 bars.

Le qualificatif de haute pression est relatif car il peut couvrir une large gamme de pressions depuis quelques bars jusqu'à la résistance de l'enceinte.

L'enceinte haute pression 2 est mise sous pression au moyen d'un dispositif de pressurisation comprenant une source d'un fluide gazeux sous pression 4 qui fournit un gaz de préférence neutre ou inerte ou de l'air stérile sous pression pénétrant dans l'enceinte haute pression 2 pour augmenter sa pression interne et un dispositif de régulation de la pression 5 qui régule la pression à l'intérieur de l'enceinte haute pression 2.

L'enceinte haute pression 2 est étanche et prévue pour résister aux hautes pressions. Elle est préférentiellement conçue pour garantir une double isolation électrique. Elle contient des poches souples 6 d'un liquide de qualité médicale 7 électriquement isolées tandis qu'une couche ou une plaque d'isolant électrique 8 est prévue sur ou dans la paroi 9 de l'enceinte haute pression 2. Cette couche d'isolant électrique 8 est préférentiellement présente sur l'ensemble de la paroi interne 10 de l'enceinte haute pression 2.

L'enceinte haute pression 2 peut être chauffée et thermostatée, de préférence à environ 37°C, pour que le liquide pulsé 11 produit par le générateur de jets pulsés haute pression 1 soit sensiblement à la température du corps humain. La régulation de cette température se fait de manière classique, de préférence par des moyens de chauffage et de régulation (non représentés) ne présentant pas de risque de souillure ou de contamination.

La source de gaz ou d'air sous pression 4 peut par exemple provenir d'un réseau de gaz ou d'air comprimé ou d'une bouteille de gaz comprimé 12. Le gaz est préférentiellement un gaz stérile inerte, chimiquement et biologiquement neutre, et respectueux de l'environnement ou de l'air stérile. Il s'agit de préférence d'azote médical.

La pression est mesurée à l'intérieur de l'enceinte haute pression 2 par un capteur de pression 13 relié si nécessaire à un transducteur de pression et contrôlée au moyen d'un circuit de contrôle 14, par exemple intégré sous la forme d'une carte électronique 15. La régulation de la pression à l'intérieur de l'enceinte haute pression 2 se fait préférentiellement soit par la commande du réglage du débit de la source de gaz sous pression 4, soit par une commande d'une purge 16 permettant de libérer le gaz ou l'air sous pression contenu dans l'enceinte 2 vers l'extérieur, soit par la combinaison de ces deux commandes. La purge 16 permettant de libérer le gaz sous pression contenu dans l'enceinte 2 vers l'extérieur peut se présenter sous la forme d'électrovannes d'ouverture et de fermeture.

De manière connue, pour réguler la pression à l'intérieur de l'enceinte haute pression 2, on utilise préférentiellement un ensemble intégré 17 sous la forme d'un bloc ou barreau et comprenant habituellement une électrovanne reliée à une vis de type BANJO, une électrovanne de sécurité normalement ouverte, une électrovanne de sécurité normalement fermée, une valve de sécurité et un manomètre-détendeur.

La plage des pressions couramment utilisées dans l'enceinte haute pression 2 s'étend de préférence entre 5 et 30 bars, par exemple égale à, ou autour de, 20 bars, car il s'agit de la zone des valeurs de pression d'un jet de liquide 11 nécessaires à la dissection ou à l'ablation de tissus. Bien évidemment, l'homme du métier pourra utiliser des pressions différentes.

La pression utilisée dans l'enceinte haute pression 2 détermine la pression du jet pulsé 11 et donc l'intensité maximale des pulsations du jet sous pression 11.

En cas de besoin, divers dispositifs hydrauliques peuvent être prévus sur les conduits et tubes intérieurs pour abaisser la pression du jet pulsé 11 sortant du générateur 1.

La chaîne hydraulique interne se compose à partir de la ou des poche(s) souple(s) d'un conduit intérieur de raccordement 18 aboutissant à un séquenceur, d'un tube amont 19 suivi d'un tube aval 20 se terminant à l'intérieur de l'enceinte par un conduit de sortie 21 haute pression.

L'alimentation à haute pression de la ou des pièce(s) à main ou d'un cathéter s'effectue par l'intermédiaire d'un conduit extérieur de liaison 22.

L'étanchéité de l'enceinte 2 au niveau du conduit de sortie haute pression 21, mais aussi du conduit d'entrée du gaz inerte de pression est préférentiellement assurée par des raccords à cône d'étanchéité tels que 23 dont la partie effilée est dirigée vers l'extérieur de l'enceinte 2 de sorte que la pression interne de l'enceinte 2 enfonce d'autant plus chaque cône pour une étanchéité proportionnellement accrue.

Le générateur de jets pulsés haute pression 1 selon l'invention comprend également au moins un connecteur 24 équipé chacun d'un percuteur stérile 25 en communication fluidique par le conduit intérieur de raccordement 18 avec l'une des sorties 26 d'une des poches souples 6. Le conduit intérieur de raccordement 18 suivant chaque connecteur 24 est également raccordé au tube amont 19. Les percuteurs 25, sont réalisés par exemple sous la forme d'une aiguille en PVC, qui est engagée dans une des sorties 26 des poches souples 6 de liquide spécial type médical 7 habituellement prévues dans ce but.

En effet, les poches souples 6 de liquide médical 7 présentent habituellement au moins une sortie 26 prévue pour les perfusions, qui sera utilisée ici pour le raccordement de la poche 6 au reste du dispositif, et une entrée 27 prévue pour l'injection d'un autre liquide dans la poche 6, qui pourra par exemple, être utilisée pour l'ajout d'un autre liquide médical 7 ou pour modifier le dosage de celui déjà présent dans la poche souple 6.

Ces connecteurs 24 de poches souples 6 peuvent également se présenter sous la forme d'un dispositif de verrouillage très simple d'utilisation qui permet de raccorder une poche souple 6 de liquide médical 7 au reste du dispositif.

Chaque connecteur 24 pour poche souple 6 peut être individuel. Il peut exister des connecteurs multiples intégrés formant un ensemble commun permettant de raccorder au moins une poche souple 6 de liquide médical à un séquenceur hydraulique dans l'enceinte haute pression 2.

Il peut s'agir pour le liquide médical contenu dans la ou les poche(s) souple(s) de liquide physiologique, de sérum, mais aussi d'un liquide médical contenant en suspension des cellules, des rétrovirus, des plasmides ou de tout agent biologique, pharmaceutique ou liquide de travail, de traitement, ou d'un liquide nécessaire à une irrigation ou à une exploration. Il peut s'agir aussi d'électrolytes ou d'un mélange de divers liquides.

Ces poches souples 6 sont préférentiellement introduites en position debout dans l'enceinte haute pression 2, leurs sorties 26 dirigées vers le haut, afin que les premiers cycles d'utilisation du générateur 1 d'impulsions d'un liquide de l'invention purgent l'air éventuellement encore présent dans les poches souples 6, ces premiers cycles d'utilisation permettant également d'effectuer un rinçage du système pour plus de sécurité avant son utilisation médicale ou chirurgicale sur un patient.

Dans le cas où les poches souples 6 de liquide médical 7 ne tiennent pas debout toutes seules dans l'enceinte haute pression 2, que ce soit par l'étroitesse de celle-ci ou par le maintien assuré par les connecteurs 24 pour poche souple 6, on peut prévoir un support individuel ou commun (non représenté) pour les poches souples 6 destiné à les maintenir en position debout, leur sorties 26 dirigées vers le haut. Un tel support permet également de maintenir les poches souples 6 en place lors des déplacements du générateur de jets pulsés haute pression 1 de l'invention.

Selon une autre variante (non représentée) de l'invention, on peut également envisager de placer les poches souples 6 de liquide médical 7 tête en bas, par exemple en les suspendant à un crochet dans la partie supérieure de l'enceinte haute pression 2 comme cela se fait de manière connue pour les perfusions.

Le coeur du générateur de jets pulsés haute pression 1 selon l'invention est un séquenceur hydraulique 28, comprenant :
- une enveloppe ou un contenant déformable souple 29 logé(e) dans l'enceinte haute pression 2 et exposé(e) à la pression intérieure qui règne dans l'enceinte ou un simple tronçon de tube souple de préférence facilement comprimable ;
- au moins un, mais de préférence et non obligatoirement deux moyens obturateurs hydrauliques commandés tels que 30 dont, s'ils sont deux, un situé de chaque côté à savoir, l'un du côté entrée et l'autre du côté sortie ou de part et d'autre de l'enveloppe souple qui est reliée hydrauliquement à l'un et à l'autre ou sont présents à chacune des extrémités du tube souple comprimable ;
- un moyen de commande alternée et cadencée des différents obturateurs hydrauliques tels que 30 ;
- un obturateur hydraulique amont 31 logé dans l'enceinte haute pression 2 et prévu entre l'extrémité du conduit intérieur de raccordement 18 et le tube amont 19. Cet obturateur hydraulique amont 31 peut être supprimé ou remplacé par un clapet anti-retour ou un clapet piloté ou équivalent comme on le verra ci-après ou dans une version très simplifiée être supprimé ;
- un obturateur hydraulique aval 32, logé dans l'enceinte haute pression 2 et prévu en aval de l'enveloppe déformable souple 29 et du tube aval 20 avant le conduit de sortie 21 ; et
- un ensemble de commande 33 agissant sur les obturateurs hydrauliques 31 et 32 de façon cadencée et ordonnée.

L'enveloppe déformable souple ou le contenant déformable souple 29 peut être de différents types avec un élargissement pour constituer un supplément de capacité ou une réserve.

Il peut s'agir d'abord d'une déformation souple en saillie par exemple en forme de bulbe dans un tube de préférence également souple.

Il peut s'agir aussi d'un composant ou d'une pièce séparée souple et déformable insérée dans la liaison hydraulique entre les deux obturateurs tel qu'un ballonnet, une poire, un bulbe ou toute autre pièce analogue. Elle se trouve alors montée en série entre les deux tubes amont et aval 19 et 20 qui peuvent alors être rigides ou semi-rigides en tout cas moins déformables que l'enveloppe souple 29 ou équivalent ou analogue.

Dans tous les cas, l'enveloppe souple 29 doit pouvoir revenir à sa forme initiale par exemple par retour élastique.

Les obturateurs hydrauliques tels que 30 sont au nombre de deux dans la version de base.

Les obturateurs hydrauliques référencés 31 et 32 peuvent être réduits à un seul. On maintient de préférence l'obturateur aval 32. Ils peuvent également être remplacés chacun ou un seul uniquement par un clapet anti-retour ou piloté ou par un composant hydraulique équivalent. On remplacera de préférence l'obturateur amont 31.

Les conduits de raccordement intérieurs 18 et les tubes d'entrée 19 et de sortie 20 du générateur d'impulsions liquides haute pression 1 sont préférentiellement en une matière plastique souple destinée à l'usage médical, tel que la silicone, le latex, le PVC, etc.

Chaque conduit de raccordement intérieur 18 et chaque tube 19, 20 du générateur d'impulsions liquides haute pression 1 peut être d'une seule pièce ou être constitué de plusieurs tubes ou conduits raccordés ensemble au moyen de raccords adaptés (non représentés).

A l'intérieur de l'enceinte haute pression 2, pour les connexions des différents conduits de raccordement intérieur 18 et tubes 19, 20 entre eux, on utilise préférentiellement des embouts biconiques stériles à usage unique.

Pour des raisons sanitaires évidentes, les différents conduits 18 et tubes 19, 20 doivent être stériles. On utilise de préférence des conduits 18 et tubes 19, 20 à usage unique qui sont remplacés après chaque utilisation du générateur de jets pulsés haute pression 1 selon l'invention.

L'ensemble peut être remplacé par une cassette qui intègre les différents composants et qui, constituant une unité fonctionnelle unique, est totalement remplaçable et interchangeable.

Pour le bon fonctionnement du générateur d'impulsions liquides moyenne et haute pression 1, à l'intérieur de l'enceinte haute pression 2, il est cependant préféré pour des raisons de précision dans les quantités, d'utiliser des conduits 18 et tubes 19, 20 en une matière présentant une rigidité supérieure à celle de l'enveloppe déformable souple 29, notamment afin d'éviter qu'un conduit 18 ou tube 19, 20 situé à l'intérieur de l'enceinte haute pression 2 ne se déforme au cours du fonctionnement du générateur.

Pour le conduit d'entrée relié à la source de gaz sous pression 4 et pour le conduit extérieur de liaison 22, on peut utiliser des conduits classiques connus de l'homme du métier, adaptés respectivement à la circulation d'un gaz sous pression et à l'alimentation en liquide sous pression pour une pièce à main.

Comme indiqué, l'enveloppe déformable souple 29 se présente à titre d'exemple sous la forme d'un bulbe ou d'un ballonnet souple déformable. Elle est préférentiellement préformée, pour qu'une fois vidée partiellement ou totalement de son contenu, elle reprenne automatiquement sa forme initiale et son volume en créant un effet d'aspiration, ce qui permet de la remplir à nouveau lorsque les obturateurs hydrauliques 30 situés en amont sont ouverts ou automatiquement dans le cas d'une liaison amont sans obturateur.

L'enveloppe déformable souple 29 présente un volume constant et dosé, préférentiellement un volume compris entre 200 • l et 1 ml, ce volume correspondant à la quantité maximale de liquide pouvant être libéré à chaque séquence de jet pulsé 11 produit par le générateur de jets pulsés haute pression 1 de l'invention. Selon l'application envisagée, l'enveloppe déformable souple 29 peut bien entendu présenter un volume différent.

L'enveloppe déformable souple 29 peut également se présenter sous la forme d'un simple tronçon de tube très souple et facilement déformable, par exemple en latex, dont le volume est prédéterminé et correspond à celui nécessaire à chaque séquence ou train d'impulsions de jet pulsé 11 produit par le générateur 1.

Selon une variante de l'invention, l'enveloppe déformable souple 29 peut également présenter un volume supérieur à celui correspondant à la quantité de liquide libéré à chaque séquence d'impulsions de jet pulsé 11 produit par le générateur de jets pulsés moyenne et haute pression 1 de l'invention. Dans ce cas, l'obturateur hydraulique aval 32 du séquenceur hydraulique 28 agit sur le tube de sortie 20 de l'enveloppe déformable souple 29 pour fractionner le volume de liquide sous pression contenu dans l'enveloppe déformable souple 29, et ceci par exemple en une ou plusieurs quantités de liquide libérées à chaque impulsion ou séquence d'impulsions de jet pulsé 11. La durée et la périodicité des pulsations du jet sous pression 11 dépendent alors directement de la durée et la périodicité des phases d'ouverture et de fermeture de l'obturateur hydraulique aval 32 du séquenceur hydraulique 28 monté sur le, ou à l'extrémité du, tube de sortie 20 de l'enveloppe déformable souple 29.

L'enveloppe déformable souple 29 peut être amovible et donc interchangeable. Elle doit être stérile. Aussi peut-elle être à usage unique et être remplacée après chaque utilisation du générateur de jets pulsés haute pression 1 selon l'invention ou être spécifique à une application donnée. Ainsi, une enveloppe déformable souple 29 spécifique à un volume différent peut être prévue pour chaque application envisagée et insérée en ligne entre les obturateurs hydrauliques, 31 et 32 pour permettre d'injecter le volume souhaité.

Un obturateur hydraulique supplémentaire 34 peut être prévu en amont de l'enveloppe déformable souple 29 comme représenté sur la figure 2, celui-ci étant relié hydrauliquement par un conduit de raccordement 35 à une seconde poche souple 36.

L'ensemble des obturateurs hydrauliques est commandé par l'ensemble de commande 33.

On notera que l'utilisation d'une seconde poche souple 36 en amont de l'enveloppe déformable souple 29 permet d'injecter par l'obturateur supplémentaire 34 un second liquide médical 37 en même temps ou alternativement avec le premier liquide médical 7 contenu dans la première poche souple 6. L'obturateur hydraulique 34 en aval de cette seconde poche 36 est commandé par le moyen de commande 33 afin d'injecter le second liquide médical 37 contenu dans celle-ci selon un dosage précis et prédéterminé par l'utilisateur.

Un autre obturateur supplémentaire (non représenté) peut être prévu en amont ou en aval de l'enveloppe déformable pour pouvoir facilement l'isoler, par exemple afin de la changer ou de la remplacer.

Chaque obturateur hydraulique tel que 30 est de préférence un élément mécanique permettant alternativement d'obturer et de libérer l'obturation du conduit 18 ou du tube 19, 20 sur lequel il est monté. Il s'agit de préférence d'obturateurs hydrauliques rapides de type tout-ou-rien, par exemple sous la forme d'électrovannes ou d'un dispositif à pincement, de moteurs à cames ou d'autres actionneurs qui pincent et libèrent alternativement les conduits 18 et tubes 19, 20 sur lesquels ils sont montés afin que les impulsions de liquide sous pression 11 produites présentent chacune une forme de signal en créneaux carrés ou rectangulaires.

Chaque obturateur hydraulique, 31, 32, 34 peut également être une électrovanne insérée dans le conduit 18 ou le tube 19, 20, un moyen mécanique de resserrement ou d'écrasement du conduit 18 ou du tube 19, 20, un clapet anti-retour ou un clapet piloté dont le rôle essentiel est d'éviter le retour du liquide 7 vers la ou les poches 6 ou 36.

De manière générale, des clapets anti-retour (non représentés) peuvent être prévus sur les divers conduits 18 et tubes 19, 20 afin d'éviter tout retour de liquide 7 vers la ou les poches 6 et tout mélange des différents liquides médicaux 7 utilisés.

Les différents moyens constitutifs du séquenceur hydraulique 28 sont préférentiellement montés sur un boîtier isolant en matière plastique. Il en est de même pour la commande 33 qui est protégée par un boîtier.

Le générateur de jets pulsés haute pression 1 selon l'invention peut aussi comprendre au moins un raccordement hydraulique prévu dans l'enceinte haute pression 2 pour la connexion d'une des sorties 26 de chacune des poches souples 6 ou 36 de liquide médical au tube amont 19 d'entrée de l'enveloppe déformable souple 29 du séquenceur hydraulique 28. Ce raccordement est préférentiellement assuré par un ou des conduit(s) de raccordement intérieur 18 et 35 à partir de chacun des connecteurs 24 de chacune des poches souples 6 ou 36, jusqu'au séquenceur 28.

Le fonctionnement du générateur de jets pulsés haute pression 1 selon l'invention est assuré par le moyen de commande 33 des différents obturateurs hydrauliques 31, 32, 34. Il s'agit de préférence d'une carte électronique pouvant être programmée par l'utilisateur ou par un logiciel pour commander de manière séquentielle ou continue l'ouverture et la fermeture des différents obturateurs hydrauliques 31, 32, 34.

En mode de fonctionnement continu du générateur de haute pression 1 de l'invention, le moyen de commande 33 contrôle et commande les ouvertures et les fermetures des obturateurs hydrauliques et au moins les mouvements des obturateurs hydrauliques amont 31 et aval 32 de part et d'autre de l'enveloppe déformable souple 29.

En mode de fonctionnement pulsé du générateur de jets pulsés 1 de l'invention, le moyen de commande 33 contrôle et commande les ouvertures d'au moins un obturateur hydraulique 31, 34 en amont de l'enveloppe déformable souple 29 et de manière séquencée et alternée l'obturateur hydraulique 32 en aval de l'enveloppe déformable souple 29 pour permettre d'abord le remplissage de l'enveloppe déformable souple 29 puis la génération d'un jet de liquide pulsé 11 dépendant du temps d'ouverture du ou des obturateurs hydrauliques 31 et 32 respectivement en amont et en aval de l'enveloppe déformable souple 29.

Toutes les possibilités de séquencement pour le jet pulsé 11 peuvent être envisagées grâce au moyen de commande 33.

Enfin, le générateur de jets pulsés haute pression 1 selon l'invention comprend en outre un moyen de raccordement étanche 39 pour le branchement du conduit de liaison 22 avec le conduit de sortie 21 du séquenceur hydraulique 28 à au moins une pièce à main 40 d'intervention médicale ou chirurgicale, ce moyen de raccordement étanche 39 étant prévu au niveau de la paroi ou à l'extérieur de l'enceinte haute pression 2.

Le moyen de raccordement étanche 39 pour pièces à main d'intervention 40 est de préférence un raccord étanche de type rapide, par exemple celui connu sous la dénomination « Luer Lock ».

Par pièce à main d'intervention 40 on considère toute pièce à main ou outil ou instrument médical ou chirurgical, endoscopique ou autre de travail ou d'exploration pouvant être raccordé au conduit de sortie 21 du séquenceur hydraulique 28 par un conduit externe de liaison 22 et utilisant le jet pulsé haute pression 11. Il s'agit préférentiellement d'une pièce à main d'intervention, par exemple un bistouri à eau, un bistouri électrique, un cathéter ou autre. Il peut bien entendu aussi, s'agir d'une pièce à main d'intervention ou d'un instrument présentant plusieurs fonctions.

Le générateur peut également être raccordé à un cathéter simple ou complexe pouvant comporter plusieurs voies et des moyens mécaniques d'intervention ainsi que des moyens de vision tels qu'une caméra miniature en vue de travaux d'exploration avec possibilité d'intervention ou de pose de prothèse d'implants ou autres.

Avantageusement, le moyen de commande 33 qui contrôle les différents obturateurs hydrauliques tels que 30, peut être associé au dispositif de régulation de la pression 5 et à celui de régulation de la température de l'enceinte sous pression, ainsi qu'à tout autre moyen constitutif de l'invention susceptible d'être contrôlé en vue d'une automatisation et/ou d'une régulation. Ainsi, toutes les commandes et régulations du générateur d'impulsions liquides haute pression 1 de l'invention sont intégrées au sein d'un même moyen de commande 5, 33 unique, plus pratique pour l'utilisateur, et plus compact.

Ce moyen de commande unique peut être logé dans l'enceinte haute pression 2 ou dans un boîtier externe 38 monté sur l'enceinte étanche 2 de l'invention.

La présente invention s'étend également à un séquenceur simplifié constitué par un tube calibré très souple par exemple un tube en silicone de degré de dureté très faible qui permet des obturations efficaces et rapides par pincement. Ce tube calibré très souple est associé à au moins un obturateur par exemple l'obturateur aval qui sera de préférence du type à pincement sur ce tube très souple.

Ce séquenceur sera un peu moins précis et donc destiné à des applications ne nécessitant pas un volume prédéfini précis de produit liquide. Ce type de séquenceur simplifié permettra un gain substantiel concernant le coût d'une unité remplaçable à usage unique assurant les mêmes fonctions dans la limite des performances techniques possibles.

De manière évidente, l'invention ne se limite pas aux modes de réalisation préférentiels décrits précédemment et représentés sur les différentes figures, l'homme du métier pouvant y apporter de nombreuses modifications et imaginer d'autres variantes sans sortir ni de la portée, ni du cadre de l'invention.

Par exemple, bien que sur les différentes figures on ait représenté à chaque fois une ou deux poches souples 6 et 36, l'invention peut être prévue pour en utiliser un nombre supérieur, en multipliant de manière adéquate le nombre nécessaire d'éléments constitutifs de l'invention.

De même, bien que l'invention n'utilise qu'une seule enveloppe déformable souple 29, l'homme du métier peut facilement adapter les moyens constitutifs de l'invention pour concevoir un générateur de jets pulsés haute pression 1 fonctionnant selon le même principe et comportant un plus grand nombre d'enveloppes déformables souples 29. Il ne s'agit dans ce cas, que d'une multiplication des moyens constitutifs principaux de l'invention.

Selon une variante de l'invention le ou les obturateur(s) hydraulique(s) 31, 32, 34 est ou sont combiné(s) à un moyen mécanique de contraction sur un tube souple interne au séquenceur 28.

Selon une autre variante non représentée de l'invention, on peut également envisager d'utiliser la pression du liquide pulsé 11 générée par le générateur de jets pulsés moyenne et haute pression 1 pour l'entraînement éventuel d'une partie mobile de la pièce à main d'intervention 40 connectée à celui-ci, pour son refroidissement, sa lubrification ou tout autre but.

Enfin, selon une autre variante non représentée de l'invention, on peut également envisager d'utiliser des conduits et tubes à double paroi dans le générateur d'impulsions liquides haute pression de l'invention, par exemple pour la génération simultanée de jets pulsés de deux liquides ou d'un liquide avec un gaz, par exemple de l'air comprimé provenant de la source d'air stérile comprimé servant à mettre l'enceinte sous haute pression ou provenant d'une autre source d'air comprimé.

## Revendications

1. Générateur de jets pulsés de moyenne et de haute pression (1) prévu pour générer des impulsions liquides haute pression à partir d'au moins un liquide médical (7, 35) contenu dans une poche souple (6, 36) placée dans une enceinte haute pression (2) étanche mise sous moyenne ou haute pression présentant au moins une sortie (26) pour liquides, le générateur comportant :
• une enceinte haute pression (2) étanche,
• un dispositif de pressurisation pour l'enceinte haute pression (2) comprenant :
- une source de gaz sous pression (4) qui fournit un gaz sous pression pénétrant dans l'enceinte haute pression (2) pour augmenter sa pression interne et
- un dispositif de régulation de la pression (5) qui régule la pression à l'intérieur de l'enceinte haute pression (2),
• au moins un connecteur (24) de jonction d'au moins une poche souple avec un conduit de raccordement (18), le connecteur (24) et le conduit (18) étant disposés dans l'enceinte haute pression (2) ;
le générateur (1) étant **caractérisé en ce qu'**il comporte :
• un séquenceur hydraulique (28), logé dans l'enceinte haute pression 2 et comprenant :
- au moins une entrée dans laquelle arrive l'au moins un conduit de raccordement (18) avec l'au moins une poche souple (6) et une sortie communiquant hydrauliquement avec l'extérieur du générateur,
- une liaison hydraulique souple comportant un tube amont (19), un tube aval (20) et une réserve tampon formée d'une enveloppe déformable souple (29) située entre le tube amont (19) et le tube aval (20), le tout étant exposé à la haute pression régnant dans l'enceinte haute pression (2),
- au moins un obturateur hydraulique (30, 32, 34) dans la liaison hydraulique souple,
- un moyen de commande (33) del'au moins un obturateurs hydrauliques (30, 31, 32, 34), et
• un moyen de raccordement étanche entre la sortie du séquenceur (28) et un conduit de liaison (22) avec une pièce à main ou un cathéter ou un instrument (40) d'utilisation.

2. Générateur de jets pulsés (1) selon la revendication 1, dans lequel le tube amont (19 et le tube aval (20) sont moins déformables que l'enveloppe déformable souple (29).

3. Générateur de jets pulsés (1) selon la revendication 1 ou 2, dans lequel l'enveloppe déformable souple (29) est une déformation en saillie de la liaison hydraulique.

4. Générateur de jets pulsés (1) selon l'une quelconque des revendications 1 à 3, dans lequel le séquenceur hydraulique (28) comprend au moins un obturateur hydraulique amont (31, 34) et un obturateur hydraulique aval (32) reliés entre eux par la liaison hydraulique souple

5. Générateur de jets pulsés (1) selon l'une quelconque des revendications 1 à 3, dans lequel le séquenceur hydraulique (28) ne comporte qu'un seul obturateur hydraulique, et dans lequel le seul obturateur hydraulique est l'obturateur hydraulique aval.

6. Générateur de jets pulsés (1) selon l'une quelconque des revendications précédentes, dans lequel l'enveloppe déformable souple (29) est un tube très souple calibré en volume.

7. Générateur de jets pulsés (1) selon l'une quelconque des revendications 1 à 6, dans lequel l'enceinte haute pression (2) comporte une couche d'isolant électrique (8) prévue sur l'ensemble de sa paroi interne (10).

8. Générateur de jets pulsés (1) selon l'une quelconque des revendications 1 à 7, dans lequel chaque connecteur (24) se présente sous la forme d'un moyen de verrouillage très simple d'utilisation qui permet de raccorder une poche souple (6) de liquide médical (7) au séquenceur hydraulique (28).

9. Générateur de jets pulsés (1) selon l'une quelconque des revendications 1 à 8, dans lequel les conduits et les tubes situés à l'intérieur de l'enceinte haute pression (2) sont dans une matière présentant une rigidité supérieure à celle de l'enveloppe déformable souple (29).

10. Générateur de jets pulsés (1) selon l'une quelconque des revendications 1 à 9, dans lequel l'enceinte haute pression (2) comporte un moyen de chauffage et de régulation de température.

11. Générateur de jets pulsés (1) selon l'une quelconque des revendications 1 à 10, dans lequel chaque obturateur hydraulique est un élément mécanique permettant et alternativement d'obturer et de libérer l'obturation d'un ou des tubes (19) ou (20) du séquenceur.

12. Générateur de jets pulsés (1) selon l'une quelconque des revendications 1 à 11, dans lequel les différents moyens constitutifs du séquenceur hydraulique (28) sont montés sur un boîtier isolant en matière plastique (37).

13. Générateur de jets pulsés (1) selon l'une quelconque des revendications 1 à 12, dans lequel le moyen de commande (33) des différents obturateurs hydrauliques est une carte électronique pouvant être programmée par l'utilisateur ou par un logiciel pour commander de manière séquentielle ou continue l'ouverture et la fermeture des différents obturateurs hydrauliques (31, 32, 34).

14. Générateur de jets pulsés (1) selon l'une quelconque des revendications 1 à 13, dans lequel l'un des obturateurs hydrauliques est comporte un clapet anti-retour.

15. Générateur de jets pulsés (1) selon l'une quelconque des revendications 1 à 14, dans lequel le séquenceur hydraulique (28) est formé d'un tube calibré très souple associé à au moins un obturateur.

## Patentansprüche

1. Mittel- und Hochdruckpulsstrahl-Generator (1) zum Erzeugen von Hochdruck-Flüssigkeitspulsen mindestens einer medizinischen Flüssigkeit (7, 35), die sich in einer weichen Tasche (6, 36) befindet, welche in einem dichten Hochdruckbehälter (2), der unter mittleren oder hohen Druck gesetzt ist, angeordnet ist und welche mindestens einen Ausgang (26) für Flüssigkeiten aufweist, wobei der Generator aufweist:
• einen dichten Hochdruckbehälter (2),
• eine Vorrichtung zum Setzen des Hochdruck-Behälters (2) unter Druck, aufweisend:
- eine unter Druck stehende Gasquelle (4), die ein unter Druck stehendes Gas bereitstellt, das in den Hochdruckbehälter (2) eindringt, um dessen Innendruck zu erhöhen, und
- eine Druckregelungsvorrichtung (5), die den Druck im Innern des Hochdruckbehälters (2) regelt,
• mindestens einen Verbinder (24) zum Verbinden mindestens einer weichen Tasche mit einer Anschlussleitung (18), wobei der Verbinder (24) und die Leitung (18) in dem Hochdruckbehälter (2) reguliert;
wobei der Generator (1) **dadurch gekennzeichnet ist, dass** er aufweist:
• eine in dem Hochdruckbehälter (2) angeordnete hydraulische Taktkette (28) mit
- mindestens einem Eingang, an welchem die mindestens eine Anschlussleitung (18), an der die mindestens eine weiche Tasche (6) angeschlossen ist, ankommt, und einem Ausgang, der in hydraulischer Verbindung mit der äußeren Umgebung des Generators steht,
- einer weichen Hydraulikverbindung aufweisend eine stromaufwärts angeordnete Röhre (19), eine stromabwärts angeordnete Röhre (20) und einen von einer weichen verformbaren Hülle (29) gebildeten Pufferreservoir, das zwischen der stromaufwärts angeordneten Röhre (19) und der stromabwärts angeordneten Röhre (20) angeordnet ist, wobei das Ganze dem hohen Druck, der in dem Hochdruckbehälter (2) herrscht, ausgesetzt ist,
- mindestens einer hydraulischen Schließvorrichtung (30, 32, 34) in der weichen Hydraulikverbindung,
- einer Steuereinrichtung (33) zur Steuerung der mindestens einen hydraulischen Schließvorrichtung (30, 31, 32, 34) und
• eine dichte Anschlusseinrichtung zwischen dem Ausgang der Taktkette (28) und einer mit einem Handstück oder einem Katheter oder einem Verwendungsinstrument (40) verbindenden Verbindungsleitung (22).

2. Pulsstrahl-Generator (1) nach Anspruch 1, in welchem die stromaufwärts angeordnete Röhre (19) und die stromabwärts angeordnete Röhre (20) weniger verformbar sind als die weiche verformbare Hülle (29).

3. Pulsstrahl-Generator (1) nach Anspruch 1 oder 2, in welchem die weiche verformbare Hülle (29) eine Verformung ist, die von der hydraulischen Verbindung vorsteht.

4. Pulsstrahl-Generator (1) nach einem der Ansprüche 1 bis 3, in welchem die hydraulische Taktkette (28) mindestens eine stromaufwärts angeordnete hydraulische Schließvorrichtung (31, 34) und eine stromabwärts angeordnete hydraulische Schließvorrichtung (32) aufweist, die durch die weiche Hydraulikverbindung miteinander verbunden sind.

5. Pulsstrahl-Generator (1) nach einem der Ansprüche 1 bis 3, in welchem die hydraulische Taktkette (28) nur eine einzige hydraulische Schließvorrichtung aufweist und in welchem die einzige hydraulische Schließvorrichtung die stromabwärts angeordnete hydraulische Schließvorrichtung ist.

6. Pulsstrahl-Generator (1) nach einem der vorstehenden Ansprüche, in welchem die weiche verformbare Hülle (29) eine sehr weiche Röhre mit kalibriertem Volumen ist.

7. Pulsstrahl-Generator (1) nach einem der Ansprüche 1 bis 6, in welchem der Hochdruckbehälter (2) an seiner gesamten Innenwand (10) eine elektrische Isolationsschicht (8) aufweist.

8. Pulsstrahl-Generator (1) nach einem der Ansprüche 1 bis 7, in welchem jeder Verbinder (24) in Form einer sehr leicht zu bedienenden Verriegelungseinrichtung vorliegt, die erlaubt, eine weiche Tasche (6) für eine medizinische Flüssigkeit (7), an die hydraulische Taktkette (28) anzuschließen.

9. Pulsstrahl-Generator (1) nach einem der Ansprüche 1 bis 8, in welchem die Leitungen und die Röhren, die sich im Innern des Hochdruckbehälters (2) befinden, aus einem Material bestehen, das eine höhere Steifheit hat als dasjenige der weichen verformbaren Hülle (29).

10. Pulsstrahl-Generator (1) nach einem der Ansprüche 1 bis 9, in welchem der Hochdruckbehälter (2) eine Heiz- und Temperaturregulierungseinrichtung aufweist.

11. Pulsstrahl-Generator (1) nach einem der Ansprüche 1 bis 10, in welchem jede hydraulische Schließvorrichtung ein mechanisches Element ist, das erlaubt, die eine oder mehreren Röhren (19) oder (20) der Taktkette abwechselnd zu schließen und freizugeben.

12. Pulsstrahl-Generator (1) nach einem der Ansprüche 1 bis 11, in welchem die verschiedenen Komponenten der hydraulischen Taktkette (28) an einem isolierenden Gehäuse aus Kunststoffmaterial (37) angebracht sind.

13. Pulsstrahl-Generator (1) nach einem der Ansprüche 1 bis 12, in welchem die Steuereinrichtung (33) zum Steuern der verschiedenen hydraulischen Schließvorrichtungen eine elektronische Schaltkarte ist, die von einem Benutzer oder einer Software programmiert werden kann, um auf eine sequentielle oder kontinuierliche Weise das Öffnen und Schließen der verschiedenen hydraulischen Schließvorrichtungen (31, 32, 34) zu steuern.

14. Pulsstrahl-Generator (1) nach einem der Ansprüche 1 bis 13, in welchem eine der hydraulischen Schließvorrichtungen eine Rückschlagklappe aufweist.

15. Pulsstrahl-Generator (1) nach einem der Ansprüche 1 bis 14, in welchem die hydraulische Taktkette (28) aus einer sehr weichen kalibrierten Röhre besteht, die mit mindestens einer Schließvorrichtung kombiniert ist.

## Claims

1. Generator (1) of medium and high-pressure pulsed jets designed to generate high-pressure liquid pulses of at least one medical liquid (7, 35) contained in a flexible bag (6, 36) placed in a sealed high-pressure enclosure (2) placed under medium or high pressure, having at least one outlet (26) for liquids, the generator comprising:
• a sealed high-pressure enclosure (2),
• a device for pressurising the high-pressure enclosure (2) comprising:
- a pressurised gas source (4) which supplies the high-pressure enclosure (2) with a pressurised gas in order to increase its internal pressure and
- a device (5) for regulating the pressure which regulates the pressure inside the high-pressure enclosure (2),
• at least one connector (24) for connecting at least one flexible bag to a connection conduit (18), the connector (24) and conduit (18) being disposed inside the high-pressure enclosure (2),
the generator (1) being **characterised in that** it comprises:
• a hydraulic sequencer (28) housed in the high-pressure enclosure (2) and comprising:
- at least one inlet through which the at least one conduit (18) connected to the at least one flexible bag (6) arrives and an outlet hydraulically communicating with the exterior of the generator,
- a flexible hydraulic connection comprising an upstream tube (19), a downstream tube (20) and a buffer reserve formed by a flexible deformable envelope (29) located between the upstream tube (19) and the downstream tube (20), the whole being exposed to the high pressure prevailing in the high-pressure enclosure (2),
- at least one hydraulic shut-off valve (30, 32, 34) in the flexible hydraulic connection,
- a means (33) for controlling the at least one hydraulic shut-off valve (30, 31, 32, 34) and
• a sealed connection means between the outlet of the sequencer (28) and a connecting conduit (22) to a hand piece or a catheter or an instrument (40) for use.

2. Pulsed jet generator (1) as claimed in claim 1, wherein the upstream tube (19) and the downstream tube (20) are less deformable than the flexible deformable envelope (29).

3. Pulsed jet generator (1) as claimed in claim 1 or 2, wherein the flexible deformable envelope (29) is a deformation projecting out from the hydraulic connection.

4. Pulsed jet generator (1) as claimed in any one of claims 1 to 3, wherein the hydraulic sequencer (28) comprises at least one upstream hydraulic shut-off valve (31, 34) and a downstream hydraulic shut-off valve (32) connected to one another by the flexible hydraulic connection.

5. Pulsed jet generator (1) as claimed in any one of claims 1 to 3, wherein the hydraulic sequencer (28) comprises only a single hydraulic shut-off valve and wherein the single hydraulic shut-off valve is the downstream hydraulic shut-off valve.

6. Pulsed jet generator (1) as claimed in any one of the preceding claims, wherein the flexible deformable envelope (29) is a very flexible volume-calibrated tube.

7. Pulsed jet generator (1) as claimed in any one of claims 1 to 6, wherein the high-pressure enclosure (2) comprises an electrical insulating layer (8) provided on its entire internal wall (10).

8. Pulsed jet generator (1) as claimed in any one of claims 1 to 7, wherein each connector (24) is provided in the form of a locking means that is very easy to use which enables a flexible bag (6) of medical liquid (7) to be connected to the hydraulic sequencer (28).

9. Pulsed jet generator (1) as claimed in any one of claims 1 to 8, wherein the conduits and tubes located inside the high-pressure enclosure (2) are made from a material having a higher rigidity than that of the flexible deformable envelope (29).

10. Pulsed jet generator (1) as claimed in any one of claims 1 to 9, wherein the high-pressure enclosure (2) comprises a heating and temperature regulating means.

11. Pulsed jet generator (1) as claimed in any one of claims 1 to 10, wherein each hydraulic shut-off valve is a mechanical element enabling alternate shutting off and releasing of the shut-off of one of or the tubes (19) or (20) of the sequencer.

12. Pulsed jet generator (1) as claimed in any one of claims 1 to 11, wherein the various means making up the hydraulic sequencer (28) are mounted on an insulating housing (37) made from plastic.

13. Pulsed jet generator (1) as claimed in any one of claims 1 to 12, wherein the means (33) for controlling the different hydraulic shut-off valves is an electronic card that can be programmed by the user or by software in order to control the opening and closing of the different hydraulic shut-off valves (31, 32, 34) in a sequential or continuous manner.

14. Pulsed jet generator (1) as claimed in any one of claims 1 to 13, wherein one of the hydraulic shut-off valves comprises a non-return valve.

15. Pulsed jet generator (1) as claimed in any one of claims 1 to 14, wherein the hydraulic sequencer (28) is provided in the form of a very flexible calibrated tube co-operating with at least one shut-off valve.
